# EUROPEAN PATENT APPLICATION

(11) **EP 1 256 353 A1**
(43) Date of publication of application: **13.11.2002**
(21) Application number: 01904368.6
(22) Date of filing: 09.02.2001
(51) Int. Cl.: A61K 45/00, A61K 31/435, C07D 221/04, C07D 221/20, A61P 1/02, A61P 27/02, A61P 37/00

(54) **REMEDIES FOR SJOEGREN'S SYNDROME**

(30) Priority: 10.02.2000 JP 2000033069
(71) Applicant: ONO PHARMACEUTICAL CO., LTD., Osaka-shi, Osaka 541-8526 (JP)
(72) Inventor: HAYASHI, Yoshio, Tokushima-shi, Tokushima 770-8064 (JP)
(74) Representative: Henkel, Feiler, Hänzel
(86) International application number: JP0100915
(87) International publication number: WO01058488

(57) **Abstract**

It was found that an inducible nitric oxygen synthase (i-NOS) inhibitor is effective for Sjögren's syndrome, and based on this knowledge, an agent for treating and/or preventing Sjögren's syndrome comprising the i-NOS inhibitor as an active ingredient has been completed. As the i-NOS inhibitor, for example, a condensed piperidine compound represented by formula (I), an acid addition salt thereof or a hydrate thereof is preferably used: 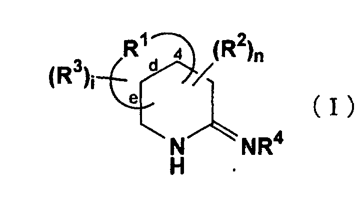 wherein symbols in the formula are described in the description.

## Description

### TECHNICAL FIELD

The present invention relates to an agent for treating Sjögren's syndrome.

More particularly, the present invention relates to an agent for treating and/or inhibiting Sjögren's syndrome comprising, as an active ingredient, an inducible nitric oxide synthase (inducible NOS; hereinafter sometimes referred to as "i-NOS") inhibitor.

### BACKGROUND ART

Sjögren's syndrome is autoimmune diseases in which xerostomia and xerokeratoconjunctivitis are main symptoms and salivary gland and lacrimal gland are target organs. As a method for treating them, administration of a nonsteroidal anti-inflammatory agent which improves immunopathy, an expectorant which stimulates exocrine gland, an eye lotion which supplements lacrimal fluid, artificial saliva or the like is carried out, but an established treatment method has not yet found. Also, since Sjögren's syndrome is autoimmune diseases, administration of an immunosuppressive agent or the like has been tried, but sufficient effects have not been obtained.

Sjögren's syndrome is one of inflammations, and it is known that an inducible nitric oxygen synthase (i-NOS) is expressed together with various inflammatory elements, interleukin-1β (IL-1β) with interferon-γ (INF-γ). Also, it is known that the inducible nitric oxygen synthase (i-NOS) is unevenly distributed in a glandular system (salivary gland, lacrimal gland) (*Curr. Eye Res*., 17(12), 1135-42 (1998), *Arthritis Rheum*., 40(5), 875-83 (1997)). However, there are many unsolved problems, for example, it is unknown which element is a main cause.

On the other hand, based on the discovery that microphages, one of immuno-competent cells, produces a large quantity of nitrate, it was found that nitric oxide (NO) was produced *in vivo* (*Proc. Natl. Acad. Sci., USA,* 82, 7738-7742 (1985), *J. Immunol*., 138, 550-565 (1987)). Also, in the field of circulatory organs, a substance having vasodilating activity released from vascular endothelial cells was found and named an endothelial derived relaxing factor (hereinafter referred to as "EDRF"). Furthermore, EDRF was found to be NO (*Nature*, 327, 524-526 (1987)).

NO which has been found to be produced *in vivo* is synthesized from L-arginine as a substrate by an nitric oxide synthase (NOS) through the following pathway:

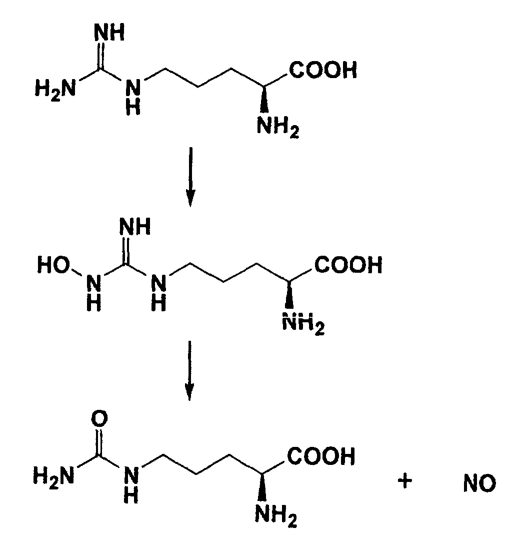

NOS includes at least a constitutive isozyme (vascular endothelial type and neuronal type) and an inducible isozyme. Vascular endothelial NOS mainly exists in vascular endothelial cells, and its activity is regulated by an intracellular calcium concentration. Neuronal NOS (constitutive NOS; hereinafter referred to as "c-NOS") exists in central nerve cells, peripheral nerve cells, islet β cells, gastrointestinal nerves, the medulla of the suprarenal glands, renal macula densa, and the like, and its activity is also regulated by the intracellular calcium concentration as well as the vascular endothelial type NOS.

The vascular endothelial type NOS and neuronal NOS (c-NOS) constantly exist in cells, and the amount of enzymes is hardly changed depending on under physiological conditions. The inducible NOS (i-NOS) exists in hepatocytes, neutrophil, macrophages, smooth muscle, fibroblasts, mesangium cells, gastrointestinal epithelium cells, islet β cells, vascular smooth cells, gliocyte, and the like. It is not found in cells and it can be induced by the stimulation of endotoxin, various cytokines and the like.

A compound represented by formula (I) which is a preferred embodiment of the active ingredient of the present invention has been filed as an inducible nitric oxygen synthase (i-NOS) inhibitor (cf. EP870763).

### DISCLOSURE OF THE INVENTION

As described above, there is no effective treatment method for Sjögren's syndrome, and since Sjögren's syndrome is autoimmune diseases, administration of an immunosuppressive agent or the like has been tried, but sufficient effects have not been obtained.

Based on the fact that expression of an inducible nitric oxygen synthase (i-NOS) in Sjögren's syndrome is limited to a glandular system, the present inventor considered that the syndrome is improved by inhibiting the inducible nitric oxygen synthase (i-NOS). Thus, dramatic effects was found in inhibition of laesio and improvement of a lacrimal gland amount by using an inducible nitric oxide synthase (i-NOS) inhibitor, and thus the present invention has been completed. It was found for the first time that the inducible nitric oxide synthase (i-NOS) inhibitor was applied to a model of Sjögren's syndrome and was effective to the model.

The inducible nitric oxygen synthase (i-NOS) inhibitor used in the present invention means all compounds having a function of inhibiting an inducible nitric oxygen synthase.

Accordingly, it includes not only known inducible nitric oxygen synthase (i-NOS) inhibitors but also inducible nitric oxygen synthase (i-NOS) inhibitors which will be found in the future. Examples include arginine analogues as substrate competitors, such as Nω-monomethyl-L-arginine (L-NMMA), Nω-nitro-L-arginine (L-NNA), Nω-amino-L-arginine (L-NAA), Nω-iminoethyl-ornithine (L-NIO) and the like. Also, examples include cofactor competitive inhibitors such as diphenylene iodonium (DPI), di-2-thienyliodonium (DTI), calcineurin and the like. However, the present invention is not limited thereto.

As the inducible nitric oxide synthase (i-NOS) inhibitor, compounds disclosed in WO96/35677, WO95/11231 WO96/14844, WO96/1484 and the like are useful, and application of these compounds to Sjögren's syndrome is also included in the present invention.

Among i-NOS inhibitor used in the present invention, a preferred embodiment is a condensed piperidine compound represented by formula (I), an acid addition salt thereof or a hydrate thereof: 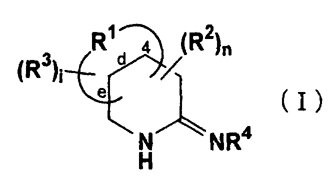 wherein -R¹- represents a 3- or 4-membered carbocyclic ring together with the carbon atom or atoms to which it is bonded, the carbocyclic ring being condensed to side d or e of the piperidine ring or bonded to the 4-position of the piperidine ring through a spiro-bond;
R² represents C1-6 alkyl;
R³ represents C1-6 alkyl, C2-6 alkenyl, C2-6 alkynyl or a halogen atom;
R⁴ represents a hydrogen atom, amino-C1-4 alkyl or carbocyclic ring-C1-4 alkyl which may be substituted with amino-C1-4 alkyl;
i represents 0 or an integer of 1 to 3;
n represents 0 or an integer of 1 to 3; and
the plural R²'s or R³'s are the same or different each other.

Among compounds represented by formula (I), a preferred compound is a condensed piperidine compound represented by formula (IA), a non-toxic salt thereof or a hydrate thereof: 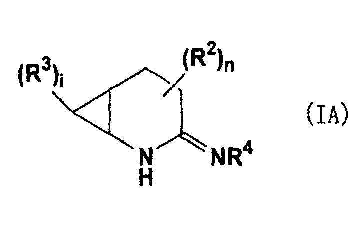 wherein symbols in the formula have the same meanings as defined above.

Among compounds represented by formula (I) and (IA), a more preferred compound is a condensed piperidine compound represented by formula (la) (i.e., (+)-trans-3-imino-5-methyl-7-chloro-2-azabicyclo[4.1.0]heptane), a non-toxic salt thereof or a hydrate thereof:

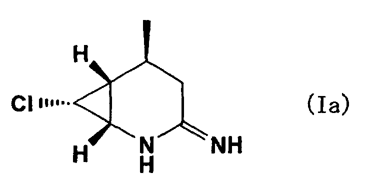

The compound used in the present invention may be administered in the form of an acid addition salt thereof. The acid addition salt is preferably non-toxic and water-soluble. Preferred acid addition salts include inorganic salts such as hydrochloride, hydrobromide, hydroiodide, sulfate, phosphate, nitrate and the like; and organic acid salts such as acetate, lactate, tartrate, oxalate, fumarate, maleate, citrate, benzoate, methanesulfonate, ethanesulfonate, benzenesulfonate, toluenesulfonate, isethionate, glucuronate, gluconate and the like. Hydrochloride is preferable.

The compound of the present invention or a salt thereof can be converted to its hydrate in a known method.

### Process for preparing the compound of the present invention:

The compound represented formula (I), (IA) or (la) of the present invention can be prepared by methods described in EP870763.

### Pharmacological activities of the compound of the present invention:

The compound represented by formula (I), (IA) or (Ia) of the present invention has a potent inducible nitric oxygen synthase (i-NOS) inhibitory activity, and as described later, is effective to a model of Sjögren's syndrome. Thus, compounds having an inducible nitric oxygen synthase (i-NOS) inhibitory activity are considered to be effective to Sjögren's syndrome.

### Toxicity:

The toxicity of the compound represented by formula (I), (IA) or (la) of the present invention is very low and it was confirmed that it is sufficiently safe as a medicament. For example, a maximum tolerated dose with an intravenous injection using the compound represented by formula (la) of the present invention is 30 mg/kg in mice.

### INDUSTRIAL APPLICABILITY

### Application to medicament:

The inducible nitric oxide synthase (i-NOS) inhibitor compound used in the present invention, an acid addition salt thereof or a hydrate thereof is effective to treatment and/or inhibition of Sjögren's syndrome.

When the inducible nitric oxide synthase (i-NOS) inhibitor compound used in the present invention, an acid addition salt thereof or a hydrate thereof is used for the above object, it is generally administered systemically or locally and orally or parenterally.

The doses to be administered changes depending on, for example, age, body weight, symptom, therapeutic effect, administration route, duration of the treatment and the like. Generally, 0.1 µg to 50 mg per adult is orally administered once to several times per day, or 0.01 µg to 50 mg per adult is parenterally administered (preferably by intravenous administration, instillation, intranasal administration, intraoral administration and the like) once to several times per day, or continuously administered from vein for 1 to 24 hours per day.

Since the dose changes depending on various conditions as described above, there are cases in which doses lower than or greater than the above ranges may be used.

The compound of the present invention may be administered as solid forms for oral administration or liquid forms for oral administration, injections, external preparations or suppositories for parenteral administration, or the like.

Solid forms for oral administration include compressed tablets, pills, capsules, dispersible powders, granules and the like. Also, they include collutories, sublingual tablets and the like which are inserted into and adhered to the oral cavity. Capsules include hard capsules and soft capsules.

In such solid forms for oral administration, one or more of the active compound(s) may used as they are or mixed with vehicles (lactose, mannitol, glucose, microcrystalline cellulose, starch, *etc.*), binders (hydroxypropyl cellulose, polyvinylpyrrolidone, magnesium metasilicate aluminate, *etc*.), disintegrants (cellulose calcium glycolate, *etc*.), lubricants (magnesium stearate, *etc.),* stabilizing agents, solution adjuvants (glutamic acid, aspartic acid, *etc*.) and the like, and formulated according to the ordinary method. Also, if necessary, the solid forms may be coated with coating agents (sugar, gelatin, hydroxypropyl cellulose or hydroxypropylmethyl cellulose phthalate, *etc*.) or be coated with two or more films. Furthermore, capsules of absorbable materials such as gelatin are also included.

Liquid forms for oral administration include pharmaceutically acceptable solutions, suspensions, emulsions, syrups, elixirs and the like. In such liquid forms, one or more of the active compound(s) may be dissolved, suspended or emulsified into generally used diluent(s) (purified water, ethanol, a mixture thereof, *etc.).* Furthermore, liquid forms may include wetting agents, suspending agents, emulsifying agents, sweetening agents, flavoring agents, aroma, preservative, buffering agent and the like.

Injections for parenteral administration include solutions, suspensions, emulsions and solid forms which are dissolved or suspended into solvent(s) for injection immediately before use. In injections, one or more of the active compound(s) may be dissolved, suspended or emulsified into solvent(s). The solvents include distilled water for injection, physiological salt solution, vegetable oil, propylene glycol, polyethylene glycol, alcohol such as ethanol, or a mixture thereof. Furthermore, injections may contain stabilizing agents, solution adjuvants (glutamic acid, aspartic acid, POLYSORBATE 80 (registered trade mark), *etc*.), suspending agents, emulsifying agents, soothing agent, buffering agents, preservatives and the like. They may be sterilized at a final step, or may be prepared and compensated according to sterile methods. They may also be manufactured in the form of sterile solid forms such as freeze-dried products, which may be dissolved in sterile water or some other sterile diluent(s) for injection immediately before use.

Other forms for parenteral administration include liquids for external use, ointments and endermic liniments, inhalations, sprays, suppositories, pessaries for vaginal administration and the like which contain one or more of the active compound(s) and which are prepared by the ordinary method.

The external liquid forms include eye lotions formulated by the ordinary method. In the same manner as the external liquid forms for parenteral administration, the eye lotions may contain stabilizing agents, solution adjuvants, suspending agents, emulsifying agents, soothing agent, buffering agents, preservatives and the like, and furthermore, may contain osmotic regulation agents (mannitol, glycerine, sodium chloride, potassium chloride, *etc*.) and thickeners (hydroxypropylmethyl cellulose, hydroxyethyl cellulose, *etc*.).

Sprays may contain stabilizing agents such as sodium sulfate, isotonic buffers such as sodium chloride, sodium citrate and citric acid, and the like, in addition to the generally used diluents. The method for preparing the sprays are described in detail in the U.S. Patents 2,868,691 and 3,095,355.

### BRIEF DESCRIPTION OF DRAWING

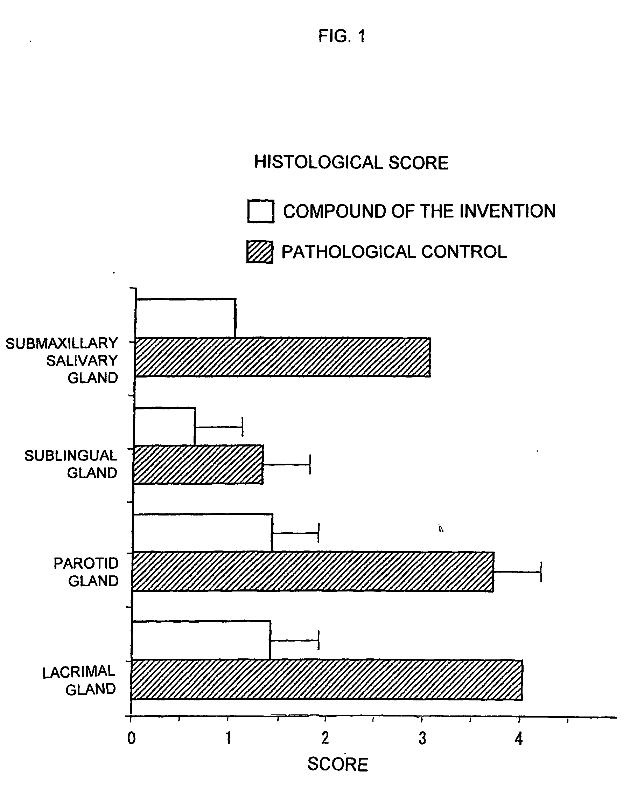

Fig. 1 is a graph showing differences in laesio between target organ (submaxillary salivary gland, sublingual gland, parotid gland, lacrimal gland) tissues of the group in which the compound of the present invention was administered and those of the non-administered group.

### BEST MODE FOR CARRYING OUT THE INVENTION

The present invention is explained in detail based on Examples, but the present invention is not limited thereto.

### Example 1

### Improvement effect of the compound of the present invention using a model mouse of naturally crisis Sjögren's syndrome:

NFS/sld female mice of which thymic gland have been extracted on the 3rd day after the birth have Sjögren's syndrome after 4 weeks *(Journal of Immunology,* 153, 2769 (1994)). The compound represented by formula (la) of the present invention was subcutaneously administered once per day (5 µg/kg/day) to the mice from 3 week age to 8 week age which were immediately before histologic grand disorder was observed. The experiment was carried out for 3 non-administered groups (pathologic control) and 5 administered groups. In the 8th week, a stimulating agent. (0.5 mg/kg pilocarpine hydrochloride) was intraperitoneally administered, and an amount of salivary gland liquid and an amount of lacrimal gland liquid were measured and evaluation was carried out by tissue findings.

### Results:

Tables 1 and 2 show amounts of gland liquids (salivary gland secretory liquid and lacrimal gland secretory liquid) as cumulative rates (AUC) which measured for 35 minutes at the interval of 5 minutes after the administration of the stimulating agent. It is demonstrated that both lacrimal gland secretory liquid and salivary gland secretory liquid were significantly improved.

**Table 1**

| | Lacrimal gland secretory liquid after administration of stimulating agent AUC_{0-35 minutes} (mm/animal weight (g)) |
|---|---|
| Group in which the compound of the present invention was administered | 0.798 |
| Pathologic control | 0.496 |

**Table 2**

| | Salivary gland secretory liquid after administration of stimulating agent AUC_{0-35 minutes} (µl/animal weight (g)) |
|---|---|
| Group in which the compound of the present invention was administered | 15.8 |
| Pathologic control | 7.41 |

As shown in the graph of Fig. 1, it is recognized that the compound of the present invention has significant inhibition effects on laesio for each target organ (submaxillary salivary gland, sublingual gland, parotid gland, lacrimal gland). The score is improved at a factor of 1/3 to 1/2 in each gland. The score was obtained based on the standard of White and Casarett (*Journal of Immunology*, 112, 178 (1974)).

### Formulation Example 1

### Preparation of tablets:

The following components were mixed and tableted by the conventional method to obtain 100 tablets each containing 1 mg of the active ingredient.

| | |
|---|---|
| (+)-Trans-3-imino-5-methyl-7-chloro-2-azabicyclo[4.1.0]heptane monohydrochloride | 100 mg |
| Carboxymethylcellulose calcium (disintegrant) | 200 mg |
| Magnesium stearate (lubricant) | 100 mg |
| Microcrystalline cellulose | 19.6g |

### Formulation Example 2

### Preparation of injection:

(+)-Trans-3-imino-5-methyl-7-chloro-2-azabicyclo[4.1.0]heptane monohydrochloride (10 mg) was dissolved into distilled water (100 ml), poured into 50 ml ampoules at 10 ml, and freeze-dried by the conventional method to obtain 10 ampoules each containing 1 mg of the active ingredient.

### Formulation Example 3

### Preparation of eye lotion:

(+)-Trans-3-imino-5-methyl-7-chloro-2-azabicyclo[4.1.0]heptane monohydrochloride (100 mg) was dissolved into distilled water (100 g), and poured into 12 ml vessels for eye lotion at 10 ml to obtain 10 eye lotions each containing 0.1% of the active ingredient.

## Claims

1. An agent for treating and/or preventing Sjögren's syndrome, comprising an inducible nitric oxygen synthase inhibitor as an active ingredient.

2. The agent for treating and/or preventing Sjögren's syndrome according to claim 1, wherein the active ingredient is a condensed piperidine compound represented by formula (I), an acid addition salt thereof or a hydrate thereof: 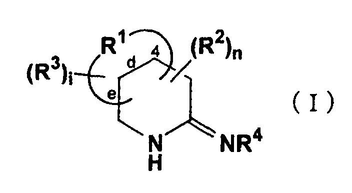 wherein -R¹- represents a 3- or 4-membered carbocyclic ring together with the carbon atom or atoms to which it is bonded, the carbocyclic ring being condensed to side d or e of the piperidine ring or bonded to the 4-position of the piperidine ring through a spiro-bond;
R² represents C1-6 alkyl;
R³ represents C1-6 alkyl, C2-6 alkenyl, C2-6 alkynyl or a halogen atom;
R⁴ represents a hydrogen atom, amino-C1-4 alkyl or carbocyclic ring-C1-4 alkyl which may be substituted with amino-C1-4 alkyl;
i represents 0 or an integer of 1 to 3;
n represents 0 or an integer of 1 to 3; and
the plural R²'s or R³'s are the same or different each other.

3. The agent for treating and/or preventing Sjögren's syndrome according to claim 2, wherein the active ingredient is a condensed piperidine compound represented by formula (IA), a non-toxic salt thereof or a hydrate thereof: 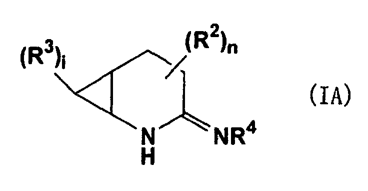 wherein symbols in the formula have the same meanings as defined in claim 2.

4. The agent for treating and/or preventing Sjögren's syndrome according to claim 3, wherein the active ingredient is a compound represented by formula (la), a non-toxic salt thereof or a hydrate thereof:

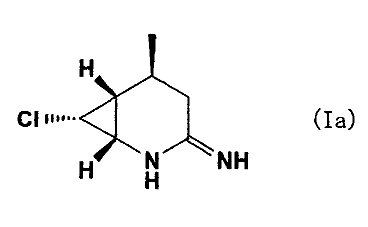

5. The agent for treating and/or preventing Sjögren's syndrome according to claim 1, wherein the active ingredient is selected from the group consisting of inducible nitric oxygen synthase inhibitor compounds described in WO96/35677, WO95/11231, WO96/14844 and WO96/14842.
